(19) Europäisches Patentamt
European Patent Office
Office européen des brevets

(11) Veröffentlichungsnummer: **0 050 585**
**B1**

(12) # EUROPÄISCHE PATENTSCHRIFT

(45) Veröffentlichungstag der Patentschrift: **03.10.84**

(51) Int. Cl.³: **C 07 D 319/20,** A 61 K 31/335

(21) Anmeldenummer: **81810403.6**

(22) Anmeldetag: **08.10.81**

(54) **1,5-Bis-(1,4-benzodioxin-2-yl)-3-azapentan-1,5-diole, Verfahren zu ihrer Herstellung, pharmazeutische Präparate enthaltend diese Verbindungen, sowie ihre therapeutische Verwendung.**

(30) Priorität: **14.10.80 US 196503**

(43) Veröffentlichungstag der Anmeldung: **28.04.82 Patentblatt 82/17**

(45) Bekanntmachung des Hinweises auf die Patenterteilung: **03.10.84 Patentblatt 84/40**

(84) Benannte Vertragsstaaten: **AT BE CH DE FR IT LI LU NL SE**

(56) Entgegenhaltungen: **JOURNAL OF MEDICINAL CHEMISTRY, Band 13, Nr. 2, März 1970 WASHINGTON (US) R. HOWE u.a. "Beta-Adrenergic blocking agents VII" Seiten 169-175**

(73) Patentinhaber: **CIBA-GEIGY AG, Postfach, CH-4002 Basel (CH)**

(72) Erfinder: **Huebner, Charles Ferdinand, Dr., 40 Edgewood Road, Chatham New Jersey (US)**
Erfinder: **Gschwend, Heinz Werner, Dr., 112 Sherwood Drive, New Providence New Jersey (US)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Gemäss dem J. Med. Chem. *13*, 169 (1970) hatte man die «Verbindung 26» der Formel I

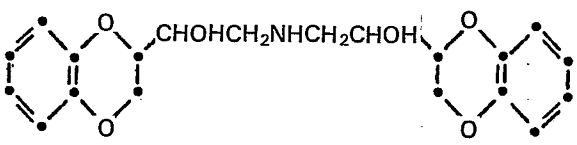

(I),

von unbestimmtem stereochemischem Aufbau, als «Nebenprodukt bei der Herstellung von Verbindungen 6 und 7», nämlich von erythro und threo «1-(1,4-Benzdioxan-2-yl)-2-amino-äthanolen», erhalten. Gemäss der genannten Publikation erhöht die «Verbindung 26» die Herzfrequenz und sie hemmt die durch Isoproterenol hervorgerufene Tachykardie.

Die Strukturformel I kann alle möglichen Stereoisomeren der erythro- und threo-Serie, nämlich die (SRSR), (RSSR), (SRRS), (RRSS), (RSRR), (SRSS), (RSSS),(SRRR), (RRRR) und (SSSS) darstellen. Die (SRSR) und (RRSS) Isomeren sind meso-Verbindungen, während die weiteren Racemate sind.

Nützt man die Vorteile der von der Anmelderin erfundenen stereospezifischen Synthesen von Zwischenprodukten aus (US-Patente 4 187 313 und 4 212 808), so können nun spezifische Isomeren der erythro- oder threo-Serie hergestellt werden. Es wurde überraschend gefunden, dass die (SRSR)-meso-Verbindung der unten angegebenen Formel II, welche der erythro-Serie angehört, ein hervorragendes antihypertensives Mittel mit bradykardischen Wirkungen ist.

Die Erfindung betrifft daher das neue spezifisch stereoisomere 1-(2,3-Dihydro-1,4-benzodioxin-2S-yl)-5-(2,3-dihydro-1,4-benzodioxin-2R-yl)-3-azapentan-1R,5S-diol der Formel II

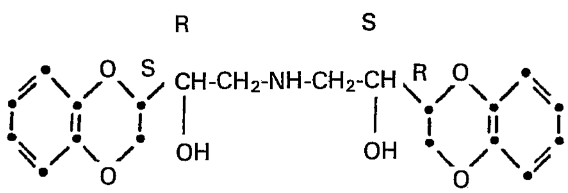

(II),

Säureadditionssalze, insbesondere therapeutisch verwendbare Säureadditionssalze dieser Verbindung, sowie Verfahren zu ihrer Herstellung, pharmazeutische Präparate enthaltend diese Verbindungen, sowie ihre therapeutische Verwendung.

Die genannten Säureadditionssalze werden vorzugsweise mit solchen Säuren oder Anionenaustauschern, welche therapeutisch verwendbare Säureadditionssalze ergeben, hergestellt. Erhaltene Salze können in die entsprechende Base der Formel II, z.B. durch Behandlung mit einer stärkeren Base, wie mit einem Metallhydroxid oder Ammoniumhydroxid, einem basischen Salz oder einen Kationenaustauscher, z.B. mit einem Alkalimetallhydroxid oder -carbonat, umgewandelt werden. Säuren, die therapeutisch verwendbare Säureadditionssalze ergeben, sind z.B. anorganische Säuren, wie Halogenwasserstoffsäuren, z.B. Chlorwasserstoff- oder Bromwasserstoffsäure, oder Schwefel-, Phosphor-, Salpeter- oder Perchlorsäure; oder vorzugsweise organische Säuren, wie aliphatische oder aromatische Carbon- oder Sulfonsäuren, z.B. Ameisen-, Essig-, Propion-, Bernstein-, Glykol-, Milch-, Äpfel-, Wein-, Zitronen-, Malein-, Hydroxymalein-, Benztrauben-, Phenylessig-, Benzoe-, 4-Aminobenzoe-, Anthranil-, 4-Hydroxybenzoe-, Salicyl-, 4-Aminosalicyl-, Pamoe-, Nikotin-, Methansulfon-, Äthansulfon-, Hydroxyäthansulfon-, Äthylensulfon-, Halogenbenzolsulfon-, Toluolsulfon-, Naphthalinsulfon-, Sulfanil- oder Cyclohexylsulfaminsäure; oder die Ascorbinsäure. Diese oder andere Salze, z.B. die Pikrate, können auch in der Reinigung der freien Base verwendet werden. Die Base wird in ihre Salze übergeführt, die Salze abgetrennt und die Base aus den Salzen freigesetzt.

Infolge der engen Beziehung zwischen der neuen Verbindung in freier Form und in Form ihrer Salze sind im vorausgegangenen und nachfolgend unter freien Verbindungen und Salzen sinn- und zweckmässig gegebenenfalls auch die entsprechenden Salze bzw. freien Verbindungen zu verstehen.

Die Verbindungen der Erfindung zeigen wertvolle pharmakologische Eigenschaften, in erster Linie antihypertensive und mässige bradykardische Wirkung. Diese pharmakologischen Eigenschaften können in Tierversuchen, vorzugsweise an Säugetieren, wie Ratten oder Hunden, als Testobjekte nachgewiesen werden. Die Tiere können vorzugsweise genetisch hypertensive Ratten oder renal hypertensive Hunde sein. Die neuen Verbindungen können ihnen enteral oder parenteral, vorzugsweise oral, z.B. durch Gelatine-Kapseln oder in Form von Stärke enthaltenden Suspensionen bzw. wässrigen Lösungen, verabreicht werden. Die verwendete Dosis kann in einem Bereich von ungefähr zwischen 0,01 und 50 mg/kg/Tag, vorzugsweise ungefähr 0,1 und 25 mg/kg/Tag, insbesondere ungefähr 1 und 10 mg/kg/Tag liegen. Die blutdrucksenkende Wirkung wird entweder direkt mit einem Katheter, der z.B. in die femulare Arterie eines Hundes eingeführt ist, oder direkt durch Sphygmomanometrie am Rattenschwanz, und einem Übertragungsinstrument registriert. Der Blutdruck wird vor und nach der Verabreichung des Wirkstoffes in mm Hg bestimmt. So sind z.B. das 1-(2,3-Dihydro-1,4-benzodioxin-2S-yl)-5-(2,3-dihydro-1,4-benzodioxin-2R-yl)-3-azapentan-1R,5S-diol-monomesylat oder das entsprechende Hydrochlorid, typische Vertreter von Verbindungen der vorliegenden Erfindung, sehr wirksame antihypertensive Mittel, wenn man sie z.B. oral an spontan hypertensive Ratten oder renal hypertensive Hunde verabreicht. Es zeigt sich in Hinsicht auf ihre antihypertensive Wirkung keine Gewöhnung bei ihrer wiederholten Verabreichung, und die Herabsetzung des Blutdruckes wird durch eine geringe Abnahme der Herzfrequenz begleitet. Antihypertensive Dosen rufen keine ungünstigen Nebenwirkungen in hypertensiven Versuchstieren oder im hochentwickelten Tierverhalten-Modell hervor. Zwar besitzt die genannte neue Verbindung der Erfindung eine $\beta$-Adrenorezeptor blockierende Wirkung, ihre Stärke ist jedoch geringer als diejenige des Propanolols, welches sonst keine antihypertensive Wirkung am Hund und

lediglich schwache Wirkungen an hypertensiven Ratten zeigt. Die antihypertensive Wirkung unserer neuen Verbindung scheint daher hauptsächlich auf einem anderen Mechanismus als die $\beta$-Adrenorezeptor-Blockade zu beruhen,und es liegt keine Evidenz zur Annahme eines cerebral lokalisierten Wirkungsmechanismus vor. Andererseits wird offensichtlich eine periphere Vasodilatation nahegelegt, welche folglich zu der eindrucksvollen antihypertensiven Wirksamkeit von Verbindungen der Erfindung wesentlich beiträgt. Die Verbindungen der vorliegenden Erfindung können daher als Antihypertensiva und als Bradykardie hervorrufende Mittel, z.B. zur Behandlung oder Handhabung der primären, sekundären oder chronischen Hypertonie, verwendet werden. Sie können auch als Zwischenprodukte zur Herstellung von anderen wertvollen, insbesondere von pharmakologisch wirksamen Präparaten eingesetzt werden.

Die Verbindungen der Erfindung können nach an sich bekannten Methoden, vorzugsweise dadurch hergestellt werden, dass man

1) erythro Verbindungen der Formeln III und IV

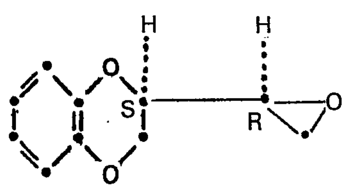

+

(III)

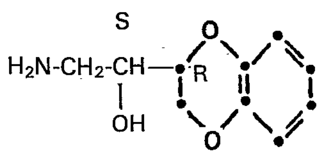

(IV)

oder jeweilige Antipoden oder Racemate davon umsetzt, und, wenn notwendig, das erhaltene Isomerengemisch auftrennt oder

2) eine Verbindung der Formel V oder das erythro Diastereomerengemisch, das eine Verbindung der Formel V

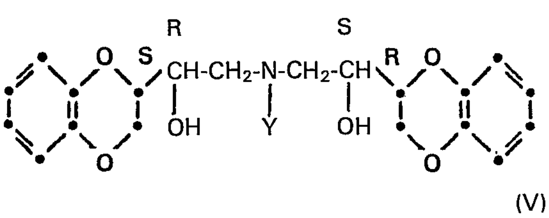

(V)

enthält, hydrogenolysiert oder hydrolysiert, worin Y einen $\alpha$-araliphatischen oder einen Acylrest bedeutet; oder Verbindungen der Formel V, worin Y für Acyl steht und eine der Hydroxygruppen mit einem Acylrest geschützt ist, hydrolysiert, und, wenn notwendig, das erhaltene Isomerengemisch auftrennt oder

3) ein d,ℓ-Gemisch einer erythro Verbindung der Formel III mit Ammoniak erhitzt, und das erhaltene Isomerengemisch auftrennt, und, wenn erwünscht, eine erhaltene Verbindung in eine andere Verbindung der Erfindung umwandelt.

Im Verfahren 1) wird die Reaktion in einem inerten Lösungsmittel, z.B. in einem aliphatischen Alkohol, vorzugsweise in einem Niederalkanol, wie Methanol, Äthanol, Propanol oder Isopropanol, bei erhöhten Temperaturen, insbesondere unter Rückflusstemperatur, durchgeführt.

Der Ausdruck «nieder» definiert in den oben oder nachfolgend genannten organischen Resten oder Verbindungen solche mit höchstens 7, vorzugsweise 4, insbesondere 1 oder 2 Kohlenstoffatomen.

In den Ausgangsstoffen der Formel V ist eine $\alpha$-araliphatische Aminoschutzgruppe Y vorzugsweise ein mono-, di- oder tricyclischer $\alpha$-araliphatischer Rest, insbesondere ein solcher $\alpha$-Aralkylrest, z.B. Benzyl, $\alpha$-Phenyläthyl, Benzhydryl oder Trityl, deren Benzolringe unsubstituierte oder durch Niederalkyl, z.B. Methyl oder Äthyl, Niederalkoxy, z.B. Methoxy oder Äthoxy, Hydroxy, Halogen, z.B. Chlor oder Brom, oder Nitro substituiert sein können. Der genannte Acylrest Y ist vorzugsweise Niederalkanoyl, z.B. Formyl, Acetyl oder Propionyl; Niederalkoxycarbonyl oder Carbobenzoxy; Carbamoyl; Sulfamoyl; aliphatisches oder aromatisches Sulfonyl, z.B. Methansulfonyl oder Toluolsulfonyl; oder Aroyl, z.B. unsubstituiertes Benzoyl oder durch Niederalkyl, Niederalkoxy oder Halogen substituiertes Benzoyl.

Je nach dem neutralen oder sauren Charakter der genannten Schutzgruppen Y, werden sie entweder durch Hydrogenolyse oder durch Hydrolyse abgespalten. Die oben erstgenannten, neutralen Schutzgruppen werden z.B. mit katalytisch aktiviertem oder nascierendem Wasserstoff, wie Wasserstoff in Gegenwart von Palladium oder mit elektrolytisch erzeugtem Wasserstoff abgespalten. Im Verfahren 2) wird die Hydrogenolyse in einem inerten Lösungsmittel, z.B. in einer Niederalkancarbonsäure oder in einem Niederalkanol, wie Essig- oder Propionsäure, oder Methanol, Äthanol, Propanol oder Isopropanol, insbesondere bei Temperaturen zwischen 10° und 75°, vorgenommen. Die oben genannten Acylverbindungen der Formel V werden vorzugsweise mit wässrigen Säuren oder Basen, oder solchen Salzen, z.B. mit Chlorwasserstoff-, Trifluoressig- oder Methansulfonsäure; oder mit wässrigen Alkalimetallhydroxiden oder -carbonaten, z.B. Natrium- oder Kaliumhydroxid oder -carbonat gespalten. Für die Hydrolyse wird als Lösungsmittel vorzugsweise ein Niederalkanol, z.B. Methanol, Äthanol, Propanol oder Isopropanol verwendet. Die Reaktion wird bei höheren Temperaturen, insbesondere beim Siedepunkt des Lösungsmittels, durchgeführt. Für die Abspaltung der oben genannten Schwefel enthaltenden Acylgruppen können auch Reduktionsmittel wie komplexe Leichtmetallhydride, z.B. Lithiumaluminiumhydrid, verwendet werden.

Im Verfahren 3) verwendet man vorzugsweise ungefähr ein halbes Moläquivalent Ammoniak. Die Reaktion wird in einem inerten Lösungsmittel, z.B. in einem aliphatischen Alkohol, vorzugsweise in einem Niederalkanol, wie Methanol, Äthanol, Propanol oder Isopropanol, bei erhöhten Temperaturen, z.B. zwischen 50° und 150° in einem Einschmelzrohr, vorgenommen.

Die Ausgangsstoffe der Formeln III und IV können vorzugsweise gemäss unserem Verfahren, durch die folgenden Reaktionsschritte erhalten werden:

a) 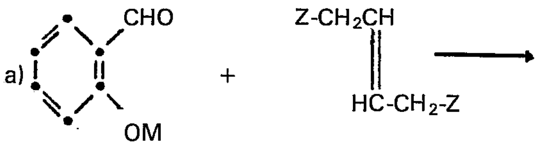

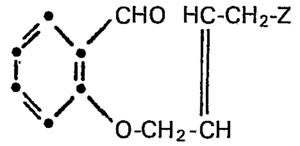 (VI),

worin M ein Alkalimetallatom bedeutet und Z für ein Halogenatom steht;

b) (VI) + 2 AcOOH→ 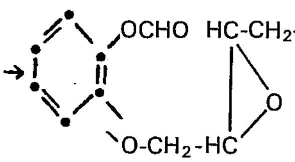 (VII),

worin der Rest Ac Niederalkanoyl, Halogenalkanoyl, unsubstituiertes oder substituiertes Benzoyl oder Phthaloyl bedeutet; und

c) Ringschluss des genannten Oxirans der Formel VII mit einem ein Hydroxylion abgebenden Mittel. Man erhält nach Auftrennung von Isomeren durch Verwendung von diasteromeren Derivaten, die Verbindung der Formel III und ihr Antipode, welche nach an sich bekannten Methoden und wie in den Beispielen gezeigt, in die Carbinolamine der Formel IV umgewandelt werden können.

Auf einem anderen Weg kann die Verbindung III durch Kondensation der folgenden Verbindungen hergestellt werden:

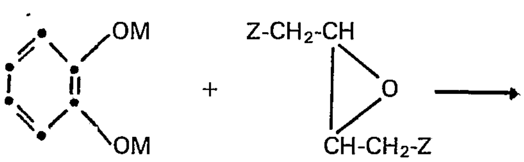 (III),

worin M und Z die oben angegebenen Bedeutungen haben. Die Reaktion wird vorzugsweise in Gegenwart von Verdünnungsmitteln, z.B. Niederalkylformamiden oder -sulfoxiden und bei Temperaturen zwischen ungefähr 50 und ungefähr 70°, durchgeführt.

Die d,ℓ-erythro-Verbindungen der Formel IV, welche man ausgehend von d,ℓ-erythro-Verbindungen der Formel III erhalten hat, und welche die R,S- und S,R-Enantiomeren enthalten, können mit optisch aktiven Säuren, z.B. Mandelsäure, Weinsäure oder Camphersulfonsäure, oder durch Herstellung von Derivaten des 1-(1-Naphthyl)-äthylisocyanats, aufgetrennt werden. Solche Trennungen können vorzugsweise wie weiter unten für die Verbindungen der Formel V beschrieben, oder gemäss J. Org. Chem. *43*, 3803 (1978), vorgenommen werden.

Der Ausgangsstoff der Formel III kann auch durch die in den Beispielen beschriebene chirale Synthese, in der man ein asymmetrisches Isocyanat zur Herstellung von chromatographisch trennbaren Gemischen verwendet, erhalten werden.

Der Ausgangsstoff der Formel V, z.B. in welchem Y Benzyl bedeutet, kann leicht hergestellt werden, indem man das erythro Diastereomerengemisch, welches man aus dem Oxiran der Formel III und seinem Antipoden (d.h. aus dem racemischen erythro Oxiran) mit dem Amin Y-$NH_2$ erhält, umkristallisiert. Vorzugsweise kristallisiert man ihre Salze, z.B. das Hydrochlorid oder Maleat um. Die Salze fallen leicht aus der Lösung aus, während die nicht-gewünschten Stereoisomeren in der Lösung bleiben. Die Umkristallisation wird vorzugsweise in wasserfreien oder wässrigen Niederalkanolen, z.B. Äthanol, n- oder Isopropanol vorgenommen. Diese Auftrennung ist ein weiterer Gegenstand unserer Erfindung, weil stereoisomere Gemische von Verbindungen mit Y=H durch Kristallisation auch dann schwer trennbar sind, wenn nur 3 Isomeren (nämlich SRSR, RSSR und SRRS) vorliegen. Das letztgenannte Gemisch von Isomeren kann chromatigraphisch aufgetrennt werden.

Ein Ausgangsstoff der Formel V, z.B. worin Y Acyl bedeutet, kann wie folgt hergestellt werden:

a) man setzt das Aminoalkohol der Formel IV, sein Racemat oder seinen Antipoden, mit einem Acylhalogenid oder einer äquivalenten Verbindung, worin Acyl vorzugsweise Niederalkanoyl, Benzoyl oder durch Niederalkyl, Niederalkoxy oder Halogen substituiertes Benzoyl bedeutet, um,

b) behandelt das erhaltene erythro 2-(2-Acylamino-1-hydroxyäthyl)-2,3-dihydro-1,4-benzodioxin mit einem Dehydratisierungsmittel, z.B. Thionylchlorid in Pyridin,

c) setzt das erhaltene erythro 2-(2-Aryl oder Niederalkyl-4,5-dihydro-oxazol-5-yl)-2,3-dihydro-1,4--benzodioxin, worin Aryl vorzugsweise Phenyl oder durch Niederalkyl, Niederalkoxy oder Halogen substituiertes Phenyl bedeutet, mit dem Oxiran der Formel III, seinem Racemat oder seinem Antipoden um.

Ausgangsstoffe und Endprodukte, welche Isomerengemische sind, können nach an sich bekannten Methoden, z.B. durch fraktionierte Destillation, Kristallisation und/oder Chromatographie, in die einzelnen Isomeren getrennt werden. Racemische Produkte können in die optischen Antipoden, z.B. bei Trennung ihrer diastereomeren Salze, z.B. durch fraktionierte Kristallisation der d- oder ℓ-Tartrate, getrennt werden.

Die oben genannten Reaktionen werden nach an sich bekannten Methoden, in Gegenwart oder Abwesenheit von Verdünnungsmitteln, vorzugsweise in solchen, welche gegenüber den Reagenzien inert sind und diese lösen, Katalysatoren, Kondensations- oder anderen oben genannten Mitteln und/oder in einer inerten Atmosphäre, unter Kühlung, bei Zimmertemperatur oder bei erhöhten Temperaturen, vorzugsweise beim Siedepunkt des verwendeten Lösungsmittels, bei normalem oder erhöhtem Druck durchgeführt.

Die Erfindung betrifft ebenfalls Abänderungen des vorliegenden Verfahrens, wonach ein auf irgendeiner Stufe des Verfahrens erhaltenes Zwischenprodukt als Ausgangsmaterial verwendet wird und die verbleibenden Verfahrensschritte durchgeführt werden, oder das Verfahren auf irgendeiner Stufe abgebrochen wird, oder wonach ein Ausgangsmaterial unter den Reaktionsbedingungen gebildet, oder worin ein Ausgangsstoff in Form eines optisch reinen Antipoden verwendet wird.

Die pharmakologisch verwendbaren Verbindungen der vorliegenden Erfindung können zur Herstellung von pharmazeutischen Präparaten verwendet werden, welche eine wirksame Menge der Aktivsubstanz zusammen oder im Gemisch mit Trägerstoffen enthalten, die sich zur enteralen oder parenteralen Verabreichung eignen. Vorzugsweise verwendet man Tabletten oder Gelatinekapseln, welche den Wirkstoff zusammen mit Verdünnungsmitteln, z.B. Laktose, Dextrose, Rohrzucker, Mannitol, Sorbitol, Cellulose und/oder Glycin, und Schmiermitteln, z.B. Kieselerde, Talk, Stearinsäure oder Salze davon, wie Magnesium- oder Calciumstearat, und/oder Polyäthylenglykol, aufweisen; Tabletten enthalten ebenfalls Bindemittel, z.B. Magnesiumaluminiumsilikat, Stärke-Paste, Gelatine, Tragant, Methylcellulose, Natriumcarboxymethylcellulose und/oder Polyvinylpyrrolidon, und, wenn erwünscht, Sprengmittel, z.B. Stärken, Agar, Alginsäure oder ein Salz davon, wie Natriumalginat und/oder Brausemischungen, oder Adsorptionsmittel, Farbstoffe, Geschmackstoffe und Süssmittel. Injizierbare Präparate sind vorzugsweise isotonische wässrige Lösungen oder Suspensionen, und Suppositorien werden in erster Linie von Fettemulsionen oder -suspensionen hergestellt. Die pharmakologischen Präparate können sterilisiert sein und/oder Hilfsstoffe, z.B. Konservier-, Stabilisier-, Netz- und/oder Emulgiermittel, Löslichkeitsvermittler, Salze zur Regulierung des osmotischen Drukkes und/oder Puffer enthalten. Die vorliegenden pharmazeutischen Präparate, die, wenn erwünscht, weitere pharmakologisch wertvolle Stoffe enthalten können, werden in an sich bekannter Weise, z.B. mittels konventioneller Misch-, Granulier- oder Dragierverfahren, hergestellt und enthalten von etwa 0,1% bis etwa 90%, insbesondere von etwa 1% bis etwa 75% des Aktivstoffes. Einzeldosen für Säuger mit einem Gewicht von ungefähr 50-70 kg können zwischen ungefähr 10 und 100 mg des aktiven Bestandteils enthalten.

Die folgenden Beispiele dienen zur Illustration der Erfindung. Temperaturen werden in Celsiusgraden angegeben, und die Angaben über Teile betreffen Gewichtsteile. Wenn nicht anders definiert, wird das Eindampfen von Lösungsmitteln unter vermindertem Druck, z.B. zwischen ungefähr 15 und 100 mm/Hg, durchgeführt.

*Beispiel 1*

Ein Gemisch von 150 g 3-Benzyl-1-(2,3-dihydro-1,4-benzodioxin-2S-yl)-5-(2,3-dihydro-1,4-benzodioxin-2R-yl)-3-azapentan-1R,5S-diol, 995 ml Eisessig und 7,5 g 10%igem Palladium-auf-Kohle Katalysator wird bei 40° und 3,4 Atmosphären bis zum Aufhören der Wasserstoffaufnahme hydriert. Das Gemisch wird filtriert, das Filtrat in 2000 ml Wasser gegossen, das Gemisch auf 10° gekühlt und mit 1500 ml 28%igem Ammoniak basisch gemacht. Der erhaltene Niederschlag wird abfiltriert, viermal mit je 500 ml Wasser gewaschen und bei 40° und 6 mmHg getrocknet. Man erhält das 1-(2,3-Dihydro-1,4-benzodioxin-2S-yl)-5-(2,3-dihydro-1,4-benzodioxin-2R-yl)-3-azapentan-1R,5S-diol, welches bei 139-141° schmilzt.

Eine Suspension von 180 g der letztgenannten Verbindung in 1230 ml wasserfreiem Äthanol wird in einer Stickstoffatmosphäre auf 78° erhitzt, wobei sich die Substanz auflöst.

Die Lösung wird gerührt und über Nacht langsam auf Zimmertemperatur gekühlt. Die erhaltenen Kristalle werden abgetrennt, zweimal mit je 35 ml Äthanol gewaschen und bei 50° und 6 mmHg getrocknet. Man erhält die genannte Verbindung in kristalliner Form, die bei 143-145° schmilzt.

Man löst 148,6 g der letztgenannten Verbindung in 1500 ml wasserfreiem Äthanol und gibt zur Lösung 43 g 98%iger Methansulfonsäure bei 65°. Die Lösung wird bei dieser Temperatur drei Minuten gerührt. Das Gemisch wird dann eine Stunde gerührt und auf 20° gekühlt, filtriert, der Rückstand dreimal mit je 80 ml Äthanol gewaschen und bei 90° und 0,5 mmHg getrocknet. Man erhält das Monomesylat der oben genannten Verbindung. F. 215-217° unter Zersetzung.

Der Ausgangsstoff wird gemäss dem vorne beschriebenen neuen Verfahren wie folgt hergestellt:

Man versetzt 130 Liter (= l) Äthanol unter Rühren und Kühlen zuerst mit 12,1 kg Natrium-methoxid und dann bei 30° mit 26,5 kg Salicylaldehyd. Dann gibt man 33,3 kg trans-1,4-Dichlor-2-buten dazu und erhitzt das Gemisch 4 Stunden bei 68°. Bei ungefähr 30° wird das Gemisch mit 156 l Toluol, 187 l Wasser und 9,4 kg 36%iger Chlorwasserstoffsäure versetzt. Der erhaltene Niederschlag wird abfiltriert und mit 16 l Toluol gewaschen. Das Filtrat wird abgetrennt, die organische Schicht dreimal mit 27 l Wasser gewaschen, eingedampft, der Rückstand destilliert und die bei 170°/0,2 mmHg siedende Fraktion aufgefangen. Man erhält den 2-(4-Chlor-trans-2-butenyloxy)-benzaldehyd.

Man gibt 23,0 kg der letztgenannten Verbindung zu einer Lösung von 63,4 kg m-Chlor-perbenzoesäure in 342 l Methylenchlorid, und hält das Gemisch 90 Minuten bei 30-35°. Das Gemisch wird 48 Stunden unter Rückfluss gekocht, auf Zimmertemperatur gekühlt und bis zur negativen Kaliumjodid/Stärke-Reaktion mit 17%iger wässriger Natriumbisulfitlösung versetzt. Das Gemisch wird filtriert, der Rückstand mit 20 l Methylenchlorid gewaschen, das Filtrat abgetrennt und die organische Schicht, welche das 2-(4-Chlor-trans-2,3-epoxy-butyloxy)-phenol-formiat enthält, gesammelt.

Die organische Schicht wird mit 50 l Methylenchlorid und 125 l Methanol verdünnt und bei 20-25° mit 16,6 kg 85%igem technischem Kaliumhydroxid in 130 l Wasser versetzt. Das Gemisch wird bei der genannten Temperatur 16 Stunden gerührt, die organische Schicht abgetrennt und mit je 50 l Wasser mehrmals gewaschen, bis der pH-Wert von 6,0-7,5 erreicht wird. Die organische Schicht wird bei 40°/20-50 mmHg eingedampft und 93 kg des rohen Rückstands werden in 33 l Isopropanol bei 60-70° gelöst. Die Lösung wird auf 25-28° gekühlt, mit Kristallkeimen versetzt, stufenweise auf —8° gekühlt und bei dieser Temperatur über Nacht stehen gelassen. Der erhaltene Niederschlag wird abfiltriert, bei —10° mit 22 l Isopropanol gewaschen und unter vermindertem Druck bei Zimmertemperatur getrocknet. Man erhält das d,ℓ-erythro-2-Oxiranyl-1,4-benzodioxan, welches bei 50-53° schmilzt. Das Produkt ist ein Racemat, welches aus der Verbindung der vorne in der Beschreibung gezeigten Formel III und aus ihrem RS-Antipoden besteht.

Das genannte Zwischenprodukt lässt sich auch gemäss einem anderen, vorne beschriebenen neuen Verfahren wie folgt herstellen: Eine Lösung von 7250 g m-Chlor-perbenzoesäure in 4500 ml Chloroform wird unter Rühren bei Zimmertemperatur unter Stickstoff mit 4125 g trans-1,4-Dichlor-2-buten innerhalb 15 Minuten versetzt. Nach einer Stunde wird das Gemisch 48 Stunden unter Rückfluss gekocht und eine Stunde bei 10° gerührt. Dann wird es filtriert, der Rückstand zweimal mit je 4000 ml Chloroform gewaschen und die vereinigten Filtrate werden dreimal mit je 5000 ml 0,5-normaler, wässriger Natriumhydroxidlösung und dann dreimal mit je 5000 ml Wasser gewaschen. Das Filtrat wird getrocknet, eingedampft, der Rückstand destilliert und die bei 87-89°/20 mmHg siedende Fraktion aufgefangen. Man erhält das trans-2,3-Bis-chlormethyloxiran.

Eine Lösung von 1390 g der letztgenannten Verbindung und 1095 g Brenzcatechin in 9870 ml Dimethylsulfoxid wird, unter Rühren bei 8° in einer Stickstoffatmosphäre, mit 711 g Natriumhydroxidtabletten versetzt. Das Gemisch wird über Nacht bei Zimmertemperatur weiter gerührt und dann mit 49 000 ml Wasser schnell versetzt. Das Gemisch wird viermal mit je 10 000 ml Diäthyläther extrahiert, der Extrakt mit 8000 ml Wasser gewaschen, getrocknet und eingedampft. Das als Rückstand erhaltene Öl wird destilliert und die bei 76-102°/0,05 mmHg siedende Fraktion aufgefangen. Man erhält das d,ℓ-erythro-2-Oxiranyl-1,4-benzodioxan, d.h. das 2,3-Dihydro-2-(2S-oxiranyl)-1,4-2R-benzodioxin (und seinen RS-Antipoden), welches bei 46-48° schmilzt.

Man gibt auf einmal 2886 g der letztgenannten Verbindung zu einer Lösung von 777 g Benzylamin in 16 200 ml Isopropanol unter Rühren in einer Stickstoffatmosphäre bei Zimmertemperatur. Das Gemisch wird 5 Stunden bei 98° gerührt, auf 30° gekühlt und mit 678 ml konz. Chlorwasserstoffsäure versetzt. Das Rühren wird über Nacht bei Zimmertemperatur fortgesetzt und die erhaltene Suspension mit 4000 ml Isopropanol verdünnt. Das Gemisch wird filtriert, der Rückstand viermal mit je 2000 ml Isopropanol gewaschen und bei 50° und 6 mmHg getrocknet. Man erhält das rohe Gemisch von drei Isomeren.

Man löst 2936 g der letztgenannten Verbindung in 44 000 ml Methanol bei 65° und rührt die Lösung, unter langsamer Kühlung auf Zimmertemperatur, über Nacht in einer Stickstoffatmosphäre. Der erhaltene Niederschlag wird abgetrennt, zweimal mit je 1000 ml Methanol gewaschen und noch zweimal umkristallisiert. Man erhält das 3-Benzyl-1-(2,3-dihydro-1,4-benzodioxin-2S-yl)-5-(2,3-dihydro-1,4-benzodioxin-2R-yl)-3-azapentan-1R,5S-diol-hydrochlorid, welches bei 215-217° schmilzt.

Zu einer Suspension von 2602 g der letztgenannten Verbindung in 28 000 ml Wasser gibt man, unter Rühren bei Zimmertemperatur in einer Stickstoffatmosphäre, 4000 ml gesättigtes wässriges Ammoniumhydroxid. Nach zwei Stunden gibt man 46 000 ml Diäthyläther dazu, hört mit dem Rühren auf und trennt die organische Schicht ab. Die wässrige Phase wird noch einmal mit 15 200 ml Diäthyläther extrahiert. Die vereinigten Extrakte werden getrocknet, filtriert und eingedampft. Man erhält die entsprechende freie Base, welche bei 108-110° schmilzt.

*Beispiel 2*

Ein Gemisch von 5 g 3-Benzyl-1-(2,3-dihydro-1,4-benzodioxin-2S-yl)-5-(2,3-dihydro-1,4-benzodioxin-2R-yl)-3-azapentan-1R,5S-diol, 50 ml Essigsäure und 0,5 g 10%igem Palladium-auf-Kohle Katalysator wird bei 3,0 Atmosphären und 25° bis zur Aufnahme von einem Moläquivalent Wasserstoff hydriert. Das Gemisch wird filtriert, eingedampft, der Rückstand mit wässriger Natriumhydroxidlösung basisch gemacht und mit Methylenchlorid extrahiert. Der Extrakt wird getrocknet, eingedampft, der Rückstand in Äthanol aufgenommen und die Lösung mit äthanolischem Chlorwasserstoff angesäuert. Man erhält das 1-(2,3-Dihydro-1,4-benzodioxin-2S-yl)-5-(2,3-dihydro-1,4-benzodioxin-2R-yl)-3-azapentan-1R,5S-diol-hydrochlorid-hemihydrat, welches unter Zersetzung bei 150-152° schmilzt.

Trocknet man das Salz bei 100°, 3 Tage im Hochvakuum, so wird das wasserfreie Hydrochlorid erhalten. F. 165-166° (unter Zersetzung).

Der Ausgangsstoff wird wie folgt hergestellt: Ein Gemisch von 9 g d,ℓ-erythro-2-Oxiranyl-1,4-benzodioxan und 2,4 g Benzylamin wird 3 Stunden in einer Stickstoffatmosphäre bei 100° gerührt. Das gekühlte Gemisch wird in Isopropanol aufgenommen, mit 4-normaler äthanolischer Chlorwasserstoffsäure angesäuert und nach 24 Stunden das kristalline Gemisch von Hydrochloridsalzen des meso-und racemischen 3-Benzyl-1-(2,3-dihydro-1,4-benzodioxin-2-yl)-5-(2,3-dihydro-1,4-benzodioxin-2-yl)-3-azapentan-1,5-diols der erythro Serie abfiltriert. F. 120-150° (unter Zersetzung). Diese Hydrochloride werden in Wasser suspendiert, das Gemisch wird mit 3-normaler wässriger Natriumhydroxidlösung basisch gemacht und mit Methylenchlorid extrahiert. Der Extrakt wird getrocknet, eingedampft und der Rückstand der entsprechenden Base aus Aluminiumoxid chromatographiert. Man eluiert mit Chloroform, welches zu einem Viertel mit konzentriertem Ammoniumhydroxid gesättigt ist. Man erhält die (SRSR)-meso-Verbindung bei $R_f = 0,85$ und das (RSSR, SRRS)-Racemat bei $R_f = 0,55$.

In einer anderen praktischeren Weise verfährt man wie folgt: Man löst 14,6 g des genannten basischen Gemisches der meso-Verbindung und des Racemates in 200 ml Isopropanol und versetzt die Lösung mit 4,1 g Maleinsäure. Man lässt die Lösung zwei Wochen bei Zimmertemperatur stehen, wobei sich das kristalline Maleat der gewünschten meso-Verbindung vollständig abtrennt. Die Mutterlauge enthält das genannte racemische Salze, welches gemäss Beispiel 1 in die freien Basen umgewandelt wird.

Das genannte racemische (RSSR)- und (SRRS)-Ba-

sengemisch wird katalytisch wie oben für die (SRSR) meso-Base gezeigt, debenzyliert. Man erhält das entsprechende racemische (RSSR, SRRS) Hydrochlorid-hemihydrat, welches bei 205° unter Zersetzung schmilzt.

*Beispiel 3*

Wie am Anfang dieser Anmeldung erwähnt ist, umfasst die Formel I eigentlich «10 mögliche Stereoisomeren». Die Konfiguration des einzigen (SRSR)-optischen Isomeren der Formel II, welches die gewünschten pharmakologischen Eigenschaften hat, wird gemäss den folgenden zwei Verfahren nachgewiesen:

1) Man lässt die heisse Lösung von 1,13 g der oben beschriebenen (SRSR)-Base, 0,71 g d-10-Camphersulfonsäure und 10 ml Isopropanol auf Zimmertemperatur abkühlen. Es werden dann 1,4 g des erhaltenen kristallinen Camphersulfonats aus Isopropanol achtmal umkristallisiert. Die Base, welche man aus dem achten umkristallisierten Produkt zurückgewinnt, zeigt ein $[\alpha]_D^{25} = 0°$ (2% in DMSO) (Dimethylsulfoxid). In analoger Weise lässt man 0,88 g des oben genannten (RSSR, SRRS)-Racemates und 0,32 g d-Apfelsäure in 3 ml heissem Isopropanol auf Zimmertemperatur abkühlen. Die erhaltenen Kristalle werden zweimal aus Isopropanol umkristallisiert. Die aus diesem Salz regenerierte Base zeigt ein $[\alpha]_D^{25} = —7{,}33°$ (3% in DMF) (Dimethylformamid). So ist nachgewiesen, dass das aufspaltbare Produkt das Racemat ist und die erstgenannte Verbindung die meso-Verbindung sein muss.

2) Ein Gemisch von 0,59 g der oben genannten (SRSR)-Base, 0,23 g ℓ-1-Phenyläthylisocyanat und 5 ml Methylenchlorid wird in einer Stickstoffatmosphäre über Nacht unter Rückfluss gekocht und eingedampft. Man erhält den entsprechenden Harnstoff. Ein paralleler Versuch mit der oben genannten racemischen Base und dem ℓ-Isocyanat ergibt ein anderes Harnstoffpräparat. Die $^{13}C$-nuklear-magnetischen Resonanzspektren werden auf einem Bruker 90,52 MHz-Apparat in $CDCl_3$ mit TMS (Tetramethylsilan) als Standard gemessen. Die Signale für die zwei aliphatischen Kohlenstoffatome des Phenyläthylamin-Teils des von der meso-Verbindung abgeleiteten Harnstoffs sind zwei Einzellinien-Signale bei 52,587 und 23,179 p.p.m.

Die entsprechenden Signale des vom Racemat abgeleiteten Harnstoffs sind zwei Doppellinien-Signale bei 52,857, 52,776 bzw. 23,152 und 23,044 p.p.m. Dies ist im Einklang mit der Erwartung, dass die Reaktion einer meso-Base mit einem chiralen Isocyanat lediglich einen Harnstoff ergibt, während die Umsetzung einer racemischen Base mit einem chiralen Isocyanat zwei Harnstoffe liefert, welche diastereomere Isomeren sind.

*Beispiel 4*

Ein Gemisch von 2,0 g 2R-(1S-Hydroxy-2-aminoäthyl)-1,4-benzodioxan, 1,8 g 2S-(1R-Oxiranyl)-1,4-benzodioxan und 20 ml Isopropanol wird über Nacht unter Rückfluss gekocht.

Nach Abkühlen säuert man es mit 4-normaler äthanolischer Chlorwasserstoffsäure an und filtriert das erhaltene Hydrochlorid-Hemihydrat ab. F. 150 bis 152°. Das Produkt ist mit demjenigen des Beispiels 2 identisch $[\alpha]_D^{25} = 0°$ (2% in DMSO).

Der Ausgangsstoff wird wie folgt hergestellt: Eine Lösung von 8 g Thiophenol in 150 ml Tetrahydrofuran wird mit Eis gekühlt und unter Rühren in einer Stickstoffatmosphäre mit 4 g 50%igem Natriumhydrid in Mineralöl versetzt. Nach dem Aufhören der Wasserstoff-Entwicklung gibt man 12,8 g d,ℓ-erythro-2-Oxiranyl-1,4-benzodioxan dazu und lässt das Gemisch sich auf Zimmertemperatur erwärmen. Das Reaktionsgemisch wird zwei Stunden unter Rückfluss gekocht, abgekühlt, mit 10%iger Chlorwasserstoffsäure angesäuert und eingedampft. Der Rückstand wird in Diäthyläther aufgenommen, die Lösung mit Wasser gewaschen und eingedampft. Man erhält das d,ℓ-erythro-2-(2-Phenylthio-1-hydroxyäthyl)-1,4-benzodioxan. Dieses wird in 50 ml Toluol aufgenommen, die Lösung mit 14 g (R)-N-[1-(1-Naphthyl)-äthyl]-isocyanat und 1% (das auf die Benzodioxanverbindung berechnete Gewichtsprozent) N,N-Dimethyläthanolamin versetzt und das Gemisch sechs Stunden unter Rückfluss gekocht. Dann wird es eingedampft und der Rückstand auf neutralem Aluminiumoxid chromatographiert. Man eluiert mit Hexan-Methylenchlorid (5:1) und kontrolliert das Eluat im UV-Spektrum bei 280 $\mu$m. Die erste Fraktion wird gesammelt und eingedampft. Man erhält das N-[1R-(1-Naphthyl)-äthyl]-1S-(1,4-benzodioxan-2R-yl)-2-phenylthioäthylcarbamat. Dann wird die zweite Fraktion aus dem Chromatogramm gesammelt und eingedampft. Sie ergibt das N-[1R-(1-Naphthyl)-äthyl]-1R-(1,4-benzodioxan-2S-yl)-2-phenylthioäthylcarbamat.

Eine Lösung von 18,8 g des aus der genannten ersten Fraktion erhaltenen Produktes in 200 ml Methylenchlorid und 10,2 ml Triäthylamin wird tropfenweise mit einer Lösung von 3,4 ml Trichlorsilan in 50 ml Methylenchlorid versetzt und das Gemisch über Nacht unter Rückfluss gekocht. Es wird abgekühlt, mit gesättigter wässriger Ammoniumchloridlösung gewaschen und eingedampft. Man erhält das erythro-2R-(2-Phenylthio-1S-hydroxyäthyl)-1,4-benzodioxan.

Man löst die letztgenannte Verbindung in 60 ml Methylenchlorid und versetzt die gerührte Lösung mit 4 g Trimethyloxonium-fluoroborat. Das Rühren wird bis zur Auflösung von allen Salzen fortgesetzt (drei Stunden). Dann gibt man unter Rühren 47 ml einer 10%igen wässrigen Natriumhydroxidlösung dazu, trennt nach drei Stunden die organische Schicht ab, trocknet sie und dampft sie ein. Man erhält das gewünschte 2R-(1S-Oxiranyl)-1,4-benzodioxan, welches für die weitere Reaktion genügend rein ist. Eine gereinigte Probe kann durch Destillation des obigen Materials in einem Kugelrohr bei 130° und 0,1 mmHg erhalten werden. Dieses Epoxid schmilzt bei 65° und $[\alpha]_D^{25} = —48{,}6°$ (2% in Chloroform).

Das Produkt der zweiten Fraktion des genannten diastereomeren Carbamats (welches in der oben beschriebenen Chromatographie erhalten wird) wird in analoger Weise behandelt. Man erhält das 2S-(1R-Oxiranyl)-1,4-benzodioxan, welches nach einer oben beschriebenen Reinigung, bei 65° schmilzt und $[\alpha]_D^{25} = +48{,}8°$.

Ein Gemisch von 10 g 2R-(1S-Oxiranyl)-1,4-benzodioxan, 3 g Natriumazid und 50 ml Dimethylformamid wird bei 90° acht Stunden gerührt. Der grösste Teil des Dimethylformamids wird abdestilliert. Der Rückstand wird mit Wasser verdünnt und mit Cyclohexan extrahiert. Der Extrakt wird mit Wasser gewaschen, getrocknet, eingedampft und das erhaltene rohe Azid in 50 ml Tetrahydrofuran gelöst. Diese Lösung wird tropfenweise zu einer gerührten und gekühlten Lösung von 3 g Lithiumaluminiumhydrid in 100 ml Tetrahydrofuran gegeben. Nach der beendeten Zugabe wird das Gemisch zwei Stunden unter Rückfluss gekocht, in Eis gekühlt und durch aufeinanderfolgende Zugabe von 3 ml Wasser, 3 ml 15%iger Natriumhydroxidlösung und 9 ml Wasser zersetzt. Die anorganischen Salze werden abfiltriert, mit Diäthylähter gewaschen, das Filtrat wird getrocknet und eingedampft. Man erhält das 2R-(1S-Hydroxy-2-amminoäthyl)-1,4-benzodioxan als ein genügend reines Öl.

*Beispiel 5*

Eine Lösung von 6 g 1,3-Bisacetyl-1-(2,3-dihydro-1,4-benzodioxin-2S-yl)-5-(2,3-dihydro-1,4-benzodioxin-2R-yl)-3-azapentan-1R,5S-diol in 100 ml Isopropanol wird mit 15 ml konzentrierter Chlorwasserstoffsäure versetzt und das Gemisch 48 Stunden unter Rückfluss gekocht. Es wird eingedampft, der Rückstand mit Isopropanol trituriert, filtriert und aus Äthanol umkristallisiert. Man erhält das 1-(2,3-Dihydro-1,4-benzodioxin-2S-yl)-5-(2,3-dihydro-1,4-benzodioxin-2R-yl)-3-azapentan-1R,5S-diol-hydrochlorid-hemihydrat, welches bei 150-152° unter Zersetzung schmilzt. Das Produkt ist mit denjenigen der Beispiele 2 und 4 identisch.

Der Ausgangsstoff wird wie folgt hergestellt: Ein Gemisch von 3 g 2R-(1S-Hydroxy-2-aminoäthyl)-1,4-benzodioxan und 10 ml Pyridin wird unter Rühren bei Zimmertemperatur mit 3 ml Essigsäureanhydrid versetzt. Das Reaktionsgemisch wird nach sechs Stunden eingedampft und der Rückstand in Methylenchlorid aufgenommen. Die Lösung wird mit 5%iger Chlorwasserstoffsäure und 5%iger wässriger Natriumhydrogencarbonatlösung gewaschen, getrocknet und eingedampft.

Das als Rückstand erhaltene Bis-acetyl-Derivat wird in 15 ml Dimethylformamid gelöst und die Lösung mit 0,74 g 50%igem Natriumhydrid in Mineralöl unter Rühren bei Zimmertemperatur versetzt. Nach zwei Stunden gibt man 2,7 g 2S-(1R-Oxiranyl)-1,4-benzodioxan dazu und rührt das Gemisch 24 Stunden in einer Stickstoffatmosphäre bei Zimmertemperatur. Das Reaktionsgemisch wird dann eingedampft, der Rückstand mit Petroläther trituriert, in Wasser aufgelöst, mit verdünnter Chlorwasserstoffsäure neutralisiert und mit Methylenchlorid extrahiert. Man erhält das 1,3-Bisacetyl-1-(2,3-dihydro-1,4-benzodioxin-2S-yl)-5-(2,3-dihydro-1,4-benzodioxin-2R-yl)-3-azapentan-1R,5S-diol.

*Beispiel 6*

Eine Lösung von 5,0 g d,ℓ-erythro-2-Oxiranyl-2,3-dihydro-1,4-benzodioxin in 50 ml Äthanol und 2,0 ml konz. Ammoniumhydroxid wird in einem Einschmelzrohr drei Tage bei 100° erhitzt. Die dunkle Flüssigkeit wird unter vermindertem Druck eingedampft, der Rückstand in 50 ml Essigsäureäthylester gelöst und mit 30 ml 1-normaler Chlorwasserstoffsäure geschüttelt. Das erhaltene feste Material wird abfiltriert und an der Luft getrocknet. Man erhält das 1-(2,3-Dihydro-1,4-benzodioxin-2-yl)-5-(2,3-dihydro-1,4-benzodioxin-2-yl)-3-azabentan-1,5-diol-hydrochlorid, welches aus einem rohen Gemisch von der meso- und d,ℓ-Isomeren besteht. Das Produkt wird in die freie Base (gemäss Beispiel 2, mit 3-normaler wässriger Natriumhydroxidlösung) umgewandelt und durch Umkehrphase (reversed phase) Hochdruck-Flüssigkeitschromatographie an Silicalgel mit gebundener Cyanpropylphase gereinigt. Als Eluierungsmittel wird ein Gemisch von 80% Methanol und 20% 0,05%iger wässriger Ammoniumcarbonatlösung verwendet. Man erhält als die am schnellsten wandernde Komponente das meso-Isomere, nämlich das 1-(2,3-Dihydro-1,4-benzodioxin-2S-yl)-5-(2,3-dihydro-1,4-benzodioxin-2R-yl)-3-azapentan-1R,5S-diol, welches bei 139-141° schmilzt. Die Verbindung ist mit derjenigen des Beispiels 1 identisch.

*Beispiel 7*

Eine Lösung von 0,45 g des rohen meso- und d,ℓ-erythro-3-Benzoyl-1-(2,3-dihydro-1,4-benzodioxin-2-yl)-5-(2,3-dihydro-1,4-benzodioxin-2-yl)-3-azapentan-1,5-diols in einer Lösung von 1 g Kaliumhydroxid im 5 ml Methanol und 0,5 ml Wasser wird über Nacht unter Rückfluss gekocht. Das Reaktionsgemisch wird mit Wasser verdünnt, und das ölige Gemisch mehrmals mit 1-normaler Chlorwasserstoffsäure extrahiert. Die saure, wässrige Schicht wird mit verdünnter wässriger Natriumhydroxidlösung basisch gemacht, mit Äther extrahiert und eingedampft. Man erhält ein rohes Öl, welches in 2 ml Isopropanol gelöst und mit 4-normaler äthanolischer Chlorwasserstoffsäure versetzt wird. Der feste Niederschlag wird abfiltriert. Man erhält das 1-(2,3-Dihydro-1,4-benzodioxin-2-yl)-5-(2,3-dihydro-1,4-benzodioxin-2-yl)-3-azapentan-1,5-diol-hydrochlorid, welches ein rohes Gemisch von meso- und d,ℓ-Isomeren ist. Dieses wird in die freie Base (gemäss Beispiel 2) umgewandelt und gemäss Beispiel 6 durch Umkehrphase (reversed phase) Hochdruck-Flüssigkeitschromatographie gereinigt. Man erhält das meso-Isomere, nämlich das 1-(2,3-Dihydro-1,4-benzodioxin-2S-yl)-5-(2,3-dihydro-1,4-benzodioxin-2R-yl)-3-azapentan-1R,5S-diol. Die Verbindung ist mit derjenigen des Beispiels 1 identisch.

Der Ausgangsstoff wird wie folgt hergestellt: Eine Lösung von 12,0 g Benzylamin in 20 ml Isopropanol wird bis zum Rückfluss gekocht und tropfenweise mit 10,0 g d,ℓ-erythro-2-Oxiranyl-2,3-dihydro-1,4-benzodioxin in 40 ml Isopropanol versetzt. Die Lösung wird über Nacht unter Rückfluss gekocht und dann unter vermindertem Druck zur Trockene eingedampft. Der Rückstand wird mit Wasser versetzt und das ölige Gemisch mehrmals mit Äther extrahiert. Die Ätherlösung wird mit 3-normaler Chlorwasserstoffsäure geschüttelt, worauf sich ein Niederschlag bildet. Dieser wird abfiltriert und aus Isopropanol umkristallisiert. Man erhält das d,ℓ-erythro-2-(2-Ben-

zylamino-1-hydroxyäthyl)-2,3-dihydro-1,4-benzodioxin-hydrochlorid, welches bei 167-169° schmilzt.

Ein Gemisch von 5,0 g d,ℓ-erythro-2-(2-Benzylamino-1-hydroxyäthyl)-2,3-dihydro-1,4-benzodioxin-hydrochlorid, in 190 ml Äthanol,10 ml Wasser und 0,5 g 10%igem Palladium-auf-Kohle Katalysator wird bei 3,4 Atmosphären hydriert. Der Katalysator wird abfiltriert und das Filtrat unter vermindertem Druck zur Trockene eingedampft. Man erhält das d,ℓ-erythro-2-(2-Amino-1-hydroxyäthyl)-2,3-dihydro-1,4-benzodioxin-hydrochlorid, welches bei 227 bis 229° schmilzt. Dieses wird gemäss Beispiel 2 in die freie Base umgewandelt.

Man löst 2,5 g d,ℓ-erythro-2-(2-Amino-1-hydroxyäthyl)-2,3-dihydro-1,4-benzodioxin (freie Base) in 25 ml Pyridin und gibt der in einem Eisbad gekühlten Lösung 1,8 ml Benzylchlorid tropfenweise zu. Die Lösung wird bei Zimmertemperatur drei Tage gerührt und unter vermindertem Druck zur Trockene eingedampft. Man erhält das rohe Produkt, welches bei 95-115° schmilzt. Nach zwei Umkristallisationen aus Äthanol erhält man das d,ℓ-erythro-2-(2-Benzoylamino-1-hydroxyäthyl)-2,3-dihydro-1,4-benzodioxin, welches bei 145-148° schmilzt.

Ein Gemisch von 2,5 g d,ℓ-erythro-2-(2-Benzoylamino-1-hydroxyäthyl)-2,3-dihydro-1,4-benzodioxin und 9,2 ml Thionylchlorid wird bei Zimmertemperatur drei Minuten gerührt und dann auf dem Dampfbad zwei Minuten erhitzt. Das Reaktionsgemisch wird zur Trockene eingedampft, mit verdünntem Natriumhydroxidl bis zum pH-Wert 11 versetzt, mit Äther extrahiert und eingedampft. Das erhaltene rohe Produkt wird aus Äthanol umkristallisiert. Man erhält das d,ℓ-erythro-2-(2-Phenyl-4,5-dihydrooxazol-5-yl)-2,3-dihydro-1,4-benzodioxin, welches bei 114-115° schmilzt.

Ein Gemisch von 0,3 g d,ℓ-erythro-2-(2-Phenyl-4,5-dihydrooxazol-5-yl)-2,3-dihydro-benzodioxin und 0,19 g d,ℓ-erythro-2-Oxiranyl-2,3-dihydro-1,4-benzodioxin wird über Nacht auf 100° erhitzt. Nach Abkühlen wird das verbliebene Ausgangsmaterial durch Hinzufügen von Äther ausgefällt. Das Filtrat, welches das erythro-3-Benzoyl-1-(2,3-dihydro-1,4-benzodioxin-2-yl)-5-(2,3-dihydro-1,4-benzodioxin-2-yl)-3-azapentan-1,5-diol als Gemisch von meso- und d,ℓ-Isomeren enthält, wird zur Trockene eingedampft. Das erhaltene rohe Material wird in der obigen Hydrolyse zum gewünschten Produkt direkt verwendet.

*Beispiel 8*

Herstellung von 10 000 Tabletten mit einem Gehalt von je 5 mg der aktiven Substanz:

*Bestandteile:*

| | |
|---|---|
| 1-(2,3-Dihydro-1,4-benzodioxin-2S-yl)-5-(2,3-dihydro-1,4-benzodioxin-2R-yl)-3-azapentan-1R,5S-diol-monomesylat | 50 g |
| Milchzucker | 1157 g |
| Maisstärke | 75 g |
| Polyäthylenglykol 6000 | 75 g |
| Talkpulver | 75 g |
| Magnesiumstearat | 18 g |
| Gereinigtes Wasser | q.s. |

*Verfahren:*

Sämtliche pulverigen Bestandteile werden mit einem Sieb von 0,6 mm Maschenweite gesiebt. Dann wird der Wirkstoff mit Milchzucker, Talk, Magnesiumstearat und mit der Hälfte der Stärke in einem geeigneten Mischer vermischt. Die andere Hälfte der Stärke wird in 40 ml Wasser suspendiert und die Suspension zur siedenden Lösung von Polyäthylenglykol in 150 ml Wasser gegeben. Die erhaltene Paste wird zu den Pulvern gegeben und gegebenenfalls unter Zugabe einer weiteren Wassermenge granuliert. Das Granulat wird über Nach bei 35° getrocknet, durch ein Sieb von 1,2 mm Maschenweite getrieben und zu Tabletten mit 6,4 mm Durchmesser, welche eine Bruchrille aufweisen, gepresst.

*Beispiel 9*

Herstellung von 10 000 Kapseln mit einem Gehalt von je 20 mg der aktiven Substanz:

*Bestandteile:*

| | |
|---|---|
| 1-(2,3-Dihydro-1,4-benzodioxin-2S-yl)-5-(2,3-dihydro-1,4-benzodioxin-2R-yl)-3-azapentan-1R,5S-diol-hydrochlorid-hemihydrat | 200 g |
| Milchzucker | 1800 g |
| Talkpuder | 100 g |

*Verfahren:*

Sämtliche pulverigen Bestandteile werden mit einem Sieb von 0,6 mm Maschenweite gesiebt. Dann wird der Wirkstoff zuerst mit Talk und darauffolgend mit Milchzucker in einem geeigneten Mischer homogenisiert. Kapseln Nr. 3 werden mit je 200 mg des Gemisches mit einer Füllmaschine gefüllt.

Die gute antihypertensive Wirkung der neuen Verbindung der Formel II wird z.B. in Form ihres Hydrochlorids, an der Ratte wie folgt nachgewiesen:

Einer Gruppe von drei spontan hypertensiven Ratten wird an zwei Taqgen je eine Dosis von 10 mg/kg der obigen Verbindung peroral verabreicht. Der systolische Blutdruck der Versuchstiere wird durch Sphygmomanometrie am Rattenschwanz vor der ersten Dosis und in bestimmten Zeitintervallen nach beiden Dosen gemessen. Die nicht-narkotisierten Ratten werden in Plexiglas-Behältern in einem temperierten Raum bei 32° gehalten.

Die folgenden Resultate (Mittelwerte) hatte man nach der oralen Verabreichung von je 10 mg/kg der genannten Verbindung an zwei aufeinanderfolgenden Tagen, erhalten:

Systolische Blutdruckabnahme in mmHg, in Stunden nach der ersten Dosis:
Nach $24^h$ = —51,0; $25^h$ = —72,0; $26^h$ = —55,0; $27^h$ = —55,7 und nach $48^h$ = —56,0 mmHg.

Gemäss den obigen Resultaten ruft die Verbindung der Formel II eine stetige Blutdrucksenkung hervor.

**Patentansprüche für die Vertragsstaaten:**
BE, CH, DE, FR, IT, LI, LU, NL, SE

1. 1-(2,3-Dihydro-1,4-benzodioxin-2S-yl)-5-(2,3-dihydro-1,4-benzodioxin-2R-yl)-3-azapentan-1R,5S-diol der Formel

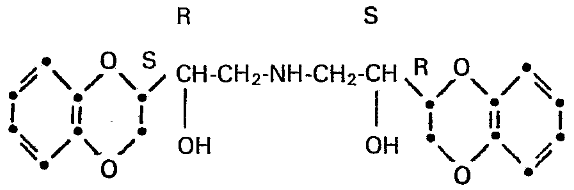

(II),

und seine Säureadditionssalze.

2. Das Hydrochlorid der im Anspruch 1 genannten Verbindung.

3. Das Methansulfonat der im Anspruch 1 genannten Verbindung.

4. Pharmazeutische Präparate enthaltend eine Verbindung gemäss einem der Ansprüche 1 bis 3, zusammen mit einem pharmazeutischen Trägermaterial.

5. Die im Anspruch 1 genannten Verbindungen zur Anwendung in einem Verfahren zur therapeutischen Behandlung des menschlichen oder tierischen Körpers.

6. Die im Anspruch 1 genannten Verbindungen als antihypertensive und bradykardische Mittel.

7. Verwendung der im Anspruch 1 genannten Verbindungen zur Herstellung von pharmazeutischen Präparaten.

8. Verfahren zur Herstellung von der im Anspruch 1 genannten Verbindung und ihren Säureadditionssalzen, dadurch gekennzeichnet, dass man

1) erythro Verbindungen der Formeln III und IV

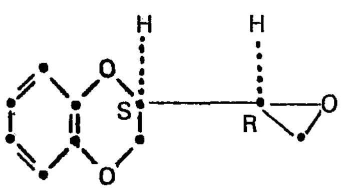

+

(III)

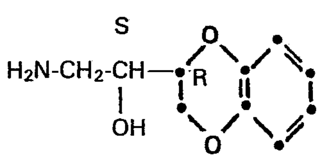

(IV)

oder jeweilige Antipoden oder Racemate davon, umsetzt, und, wenn notwendig, das erhaltene Isomerengemisch auftrennt oder

2) eine Verbindung der Formel V oder das erythro Diastereomerengemisch, das eine Verbindung der Formel V

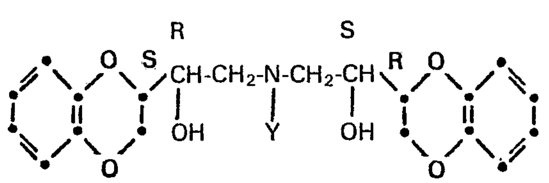

(V)

enthält, hydrogenolysiert oder hydrolysiert, worin Y einen $\alpha$-araliphatischen oder einen Acylrest bedeutet; oder Verbindungen der Formel V, worin Y für Acyl steht und eine der Hydroxygruppen mit einem Acylrest geschützt ist, hydrolysiert, und, wenn notwendig, das erhaltene Isomerengemisch auftrennt, oder

3) ein d,ℓ-Gemisch einer erythro Verbindung der Formel III mit Ammoniak erhitzt, und das erhaltene Isomerengemisch auftrennt, und, wenn erwünscht, eine erhaltene freie Verbindung in ein Säureadditionssalz oder ein erhaltenes Säureadditionssalz in die freie Verbindung oder in ein anderes Säureadditionssalz überführt.

9. Ein Isomerengemisch von meso- und d,ℓ-3-Benzyl-1-(2,3-dihydro-1,4-benzodioxin-2-yl)-5-(2,3-dihydro-1,4-benzodioxin-2-yl)-3-azapentan-1,5-diol der erythro Serie, und seine Säureadditionssalze.

10. Das 3-Benzyl-1-(2,3-dihydro-1,4-benzodioxin-2S-yl)-5-(2,3-dihydro-1,4-benzodioxin-2R-yl)-3-azapentan-1R,5S-diol und seine Säureadditionssalze.

**Patentansprüche für den Vertragsstaat:** AT

1. Verfahren zur Herstellung des neuen 1-(2,3-Dihydro-1,4-benzodioxin-2S-yl)-5-(2,3-dihydro-1,4-benzodioxin-2R-yl)-azapentan-1R,5S-diols der Formel

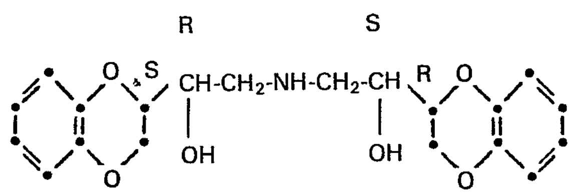

(II),

und seiner Säureadditionssalze, dadurch gekennzeichnet, dass man

1) erythro Verbindungen der Formeln III und IV

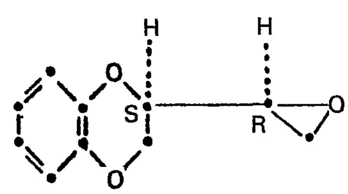

+

(III)

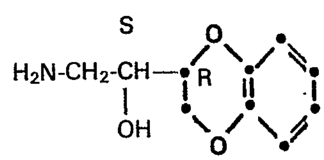

(IV)

oder jeweilige Antipoden oder Racemate davon, umsetzt, und, wenn notwendig, das erhaltene Isomerengemisch auftrennt oder

2) eine Verbindung der Formel V oder das erythro Diastereomerengmisch, das eine Verbindung der Formel V

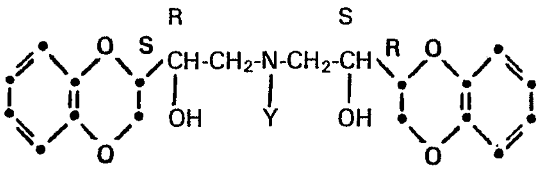

(V)

enthält, hydrogenolysiert oder hydrolysiert, worin Y einen α-araliphatischen oder einen Acylrest bedeutet; oder Verbindungen der Formel V, worin Y für Acyl steht und eine der Hydroxygruppen mit einem Acylrest geschützt ist, hydrolysiert, und, wenn notwendig, das erhaltene Isomerengemisch auftrennt, oder

3) ein d,ℓ-Gemisch einer erythro Verbindung der Formel III mit Ammoniak erhitzt, und das erhaltene Isomerengemisch auftrennt, und, wenn erwünscht, eine erhaltene freie Verbindung in ein Säureadditionssalz oder ein erhaltenes Säureadditionssalz in die freie Verbindung oder in ein anderes Säureadditionssalz überführt.

2. Verfahren nach Anspruch 1, dadurch gekennzeichnet, dass man die Reaktion 1) bei der Rückflusstemperatur des Reaktionsgemisches durchführt.

3. Verfahren nach Anspruch 1, dadurch gekennzeichnet, dass man in der Reaktion 2) einen Ausgangsstoff der Formel V, worin Y einen α-araliphatischen Rest bedeutet, durch Behandlung mit katalytisch aktiviertem oder nascierendem oder mit elektrolytisch erzeugtem Wasserstoff hydrogenolysiert.

4. Verfahren nach Anspruch 1, dadurch gekennzeichnet, dass man in der Reaktion 2) einen Ausgangsstoff, worin Y Acyl bedeutet, diesen Acylrest mit wässrigen Säuren oder Basen, oder mit komplexen Leichtmetallhydriden abspaltet.

5. Verfahren nach Anspruch 1, dadurch gekennzeichnet, dass man in der Reaktion 3) ein halbes Moläquivalent Ammoniak verwendet.

6. Verfahren nach Anspruch 1, dadurch gekennzeichnet, dass man ein erhaltenes Gemisch von Isomeren oder ihren Salzen durch Chromatographie oder Kistallisation auftrennt.

7. Verfahren nach einem der Ansprüche 1 bis 6, dadurch gekennzeichnet, dass man ein auf beliebiger Verfahrensstufte erhältliches Zwischenprodukt als Ausgangsmaterial verwendet und die übrigbleibenden Verfahrensschritte durchführt, oder einen Ausgangsstoff unter den Reaktionsbedingungen bildet oder in Form eines optisch reinen Antipoden verwendet.

8. Verfahren nach Anspruch 1 zur Herstellung des neuen 1-(2,3-Dihydro-1,4-benzodioxin-2S-yl)-5-(2,3-dihydro-1,4-benzodioxin-2R-yl)-3-azapentan-1R,5S-diols und seiner Säureadditionssalze, dadurch gekennzeichnet, dass man

1) Verbindungen der im Anspruch 1 gezeigten Formeln III und IV oder ihre Antipoden umsetzt, oder

2) eine Verbindung der im Anspruch 1 gezeigten Formel V hydrogenolysiert oder hydrolysiert, und, wenn erwünscht, eine erhaltene freie Verbindung in ein Säureadditionssalz oder ein erhaltenes Säureadditionssalz in die freie Verbindung oder in ein anderes Säureadditionssalz überführt.

9. Verfahren nach Anspruch 8, dadurch gekennzeichnet, dass man die Reaktion 1) bei der Rückflusstemperatur des Reaktionsgemisches durchführt.

10. Verfahren nach Anspruch 8, dadurch gekennzeichnet, dass man in der Reaktion 2) einen Ausgangsstoff der Formel V, worin Y einen α-araliphatischen Rest bedeutet, durch Behandlung mit katalytisch aktiviertem oder nascierendem oder mit elektrolytisch erzeugtem Wasserstoff hydrogenolysiert.

11. Verfahren nach Anspruch 8, dadurch gekennzeichnet, dass man in der Reaktion 2) einen Ausgangsstoff, worin Y Acyl bedeutet, diesen Acylrest mit wässrigen Säuren oder Basen, oder mit komplexen Leichtmetallhydriden abspaltet.

12. Verfahren nach einem der Ansprüche 8 bis 11, dadurch gekennzeichnet, dass man ein auf beliebiger Verfahrensstufe erhältliches Zwischenprodukt als Ausgangsmaterial verwendet und die übrigbleibenden Verfahrensschritte durchführt, oder einen Ausgangsstoff unter den Reaktionsbedingungen bildet oder in Form eines optisch reinen Antipoden verwendet.

13. Verfahren zur Herstellung eines pharmazeutischen Präparats, gekennzeichnet durch die Verarbeitung eines erfindungsgemässen Wirkstoffes nach einem der Ansprüche 1 bis 7, mit einem pharmazeutischen Trägermaterial.

14. Verfahren zur Herstellung eines pharmazeutischen Präparats, gekennzeichnet durch die Verarbeitung eines erfindungsgemässen Wirkstoffes nach einem der Ansprüche 8 bis 12, mit einem pharmazeutischen Trägermaterial.

**Claims for the Contracting States:**
BE, CH, DE, FR, IT, LI, LU, NL, SE

1. 1-(2,3-Dihydro-1,4-benzodioxin-2S-yl)-5-(2,3-dihydrro-1,4-benzodioxin-2R-yl)-azapentane-1R,5S-diol of the formula

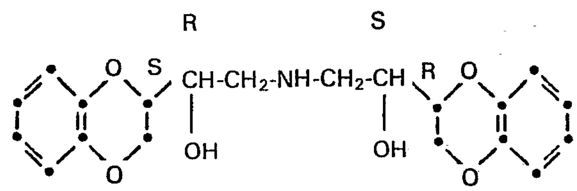

(II),

and the acid addition salts thereof.

2. The hydrochloride of the compound claimed in claim 1.

3. The methanesulfonate of the compound claimed in claim 1.

4. A pharmaceutical preparation containing a compound according to any one of claims 1 to 3, together with a pharmaceutical carrier.

5. A compound as claimed in claim 1 for use in a therapeutic method of treating the human or animal body.

6. A compound as claimed in claim 1 as antihypertensive and bradycardial agent.

7. Use of a compound as claimed in claim 1 for the manufacture of pharmaceutical preparations.

8. A process for the preparation of a compound as

claimed in claim 1 and the acid addition salts thereof, which comprises

1) reacting erythro compounds of the formula III and IV

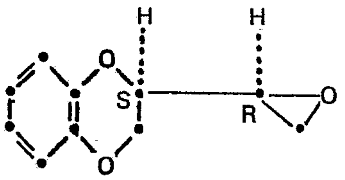

+

(III)

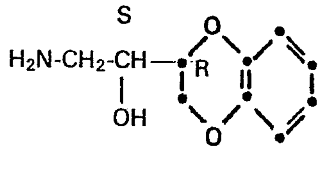

(IV)

or respective antipodes or racemates thereof, and, if necessary, separating the mixture of isomers so obtained, or

2) hydrogenolysing or hydrolysing a compound of the formula V or the mixture of erythro diasterisomers which contains a compound of the formula V

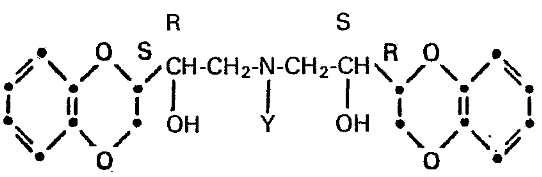

(V)

wherein Y is an $\alpha$-araliphatic radical or an acyl radical, or hydrolysing a compound of the formula V, wherein Y is acyl and one of the hydroxyl groups is protected by an acyl radical, and, if necessary, separating the mixture of isomers so obtained, or

3) heating an erythro compound of the formula III, or a racemate thereof, with ammonia and, if necessary, separating the mixture of isomers so obtained and, if desired, converting a resultant free compound into an acid addition salt or a resultant acid addition salt into the free compound or into another acid addition salt.

9. A mixture of meso- and d,ℓ-3-benzyl-1-(2,3-dihydro-1,4-benzodioxin-2-yl)-5-(2,3-dihydro-1,4-benzodioxin-2-yl)-3-azapentane-1,5-diol isomers of the erythro series, and the acid addition salts thereof.

10. 3-Benzyl-1-(2,3-dihydro-1,4-benzodioxin-2S-yl)-5-(2,3-dihydro-1,4-benzodioxin-2R-yl)-3-azapentane-1R,5S-diol and the acid addition salts thereof.

**Claims for the Contracting State:** AT

1. A process for the preparation of the novel 1-(2,3-dihydro-1,4-benzodioxin-2S-yl)-5-(2,3-dihydro-1,4-benzodioxin-2R-yl)-3-azapentane-1R,5S-diol of the formula

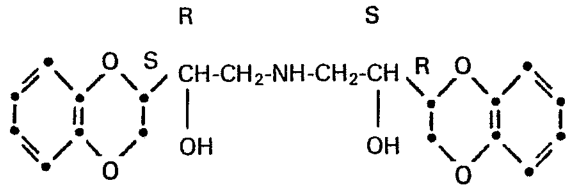

(II),

and the acid addition salts thereof, which comprises

1) reacting erythro compounds of the formulae III and IV

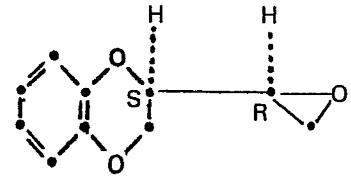

+

(III)

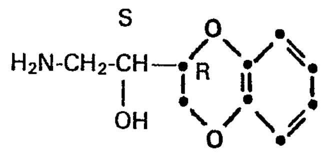

(IV)

or respective antipodes or racemates thereof, and, if necessary, separating the mixture of isomers so obtained, or

2) hydrogenolysing or hydrolysing a compound of the formula V or the mixture of erythro diasterisomers which contains a compound of the formula V

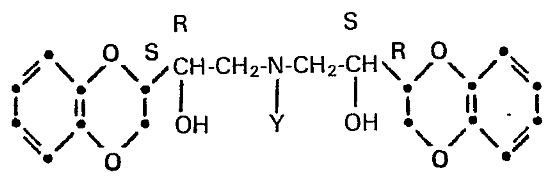

(V)

wherein Y is an $\alpha$-araliphatic radical or an acyl radical, or hydrolysing a compound of the formula V, wherein Y is acyl and one of the hydroxyl groups is protected by an acyl radical, and, if necessary, separating the mixture of isomers so obtained, or

3) heating an erythro compound of the formula III, or a racemate thereof, with ammonia and, if necessary, resolving the mixture of isomers so obtained and, if desired, converting a resultant free compound into an acid addition salt or a resultant acid addition salt into the free compound or into another acid addition salt.

2. A process according to claim 1, which comprises carrying out reaction 1) at the reflux temperature of the reaction mixture.

3. A process according to claim 1, which comprises hydrogenolysing, in reaction 2), a starting material of the formula V, wherein Y is an $\alpha$-araliphatic radical, by treatment with catalytically activated or nascent hydrogen or with electrolytically produced hydrogen.

4. A process according to claim 1, which comprises splitting off an acyl radical Y from a starting material, wherein Y is an acyl radical, in reaction 2), with an aqueous acid or base or with a complex light metal hydride.

5. A process according to claim 1, wherein a half molar equivalent of ammonia is employed in reaction 3).

6. A process according to claim 1, which comprises separating a resultant mixture of isomers or salts thereof by chromatography or crystallisation.

7. A process according to any one of claims 1 to 6, which comprises using an intermediate obtainable as starting material in any step of the process and carrying out the remaining steps, or forming a starting material under the reaction conditions, or using it in the form of an optically pure antipode.

8. A process according to claim 1 for the preparation of the novel 1-(2,3-dihydro-1,4-benzodioxin-2S-yl)-5-(2,3-dihydro-1,4-benzodioxin-2R-yl)-3-azapentane-1R,5S-diol and the acid addition salts thereof, which comprises

1) reacting compounds of the formulae III and IV as indicated in claim 1, or the antipodes thereof, or

2) hydrogenolysing or hydrolysing a compound of the formula V as indicated in claim 1 and, if desired, converting a resultant free compound into an acid addition salt or a resultant acid addition salt into the free compound or into another acid addition salt.

9. A process according to claim 8, which comprises carrying out reaction 1) at the reflux temperature of the reaction mixture.

10. A process according to claim 8, which comprises hydrogenolysing, in reaction 2), a starting material of the formula V, wherein Y is an $\alpha$-araliphatic radical, by treatment with catalytically activated or nascent hydrogen or with electrolytically produced hydrogen.

11. A process according to claim 8, which comprises splitting off an acyl radical Y from a starting material, wherein Y is an acyl radical, in reaction 2), with an aqueous acid or base or with a complex light metal hydride.

12. A process according to any one of claims 8 to 11, which comprises using an intermediate obtainable as starting material in any step of the process and carrying out the remaining steps, or forming a starting material under the reaction conditions, or using it in the form of an optically pure antipode.

13. A process for the preparation of a pharmaceutical preparation, which comprises mixing a compound according to any one of claims 1 to 7, with a pharmaceutical carrier.

14. A process for the preparation of a pharmaceutical preparation, which comprises mixing a compound according to any one of claims 8 to 12, with a pharmaceutical carrier.

**Revendications pour les Etats Contractants:**
BE, CH, DE, FR, IT, LI, LU, NL, SE

1. 1-(2,3-dihydro-1,4-benzodioxin-2S-yl)-5-(2,3-dihydro-1,4-benzodioxin-2R-yl)-3-azapentane-1R,5S-diol de formule

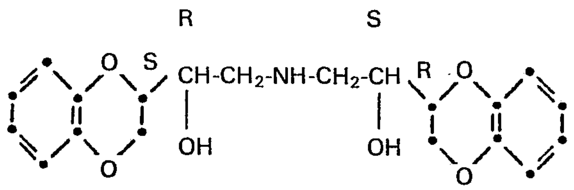

(II),

et ses sels d'addition d'acides.

2. Le chlorhydrate du composé mentionné dans la revendication 1.

3. Le méthanesulfonate du composé mentionné dans la revendication 1.

4. Préparations pharmaceutiques contenant un composé selon l'une des revendicatins 1 à 3, avec un support pharmaceutique.

5. Les composés mentionnés dans la revendication 1 aux fins d'application dans un procédé de traitement thérapeutique du corps humain ou animal.

6. Les composés mentionnés dans la revendication 1 comme agents antihypertensifs et bradycardiques.

7. Application des composés mentonnés dans la revendication 1 à la préparation de préparations pharmaceutiques.

8. Procédé de préparation du composé mentionné dans la revendication 1 et de ses sels d'addition d'acides, caractérisé en ce que

1) on fait réagir des composés érythro de formules III et IV

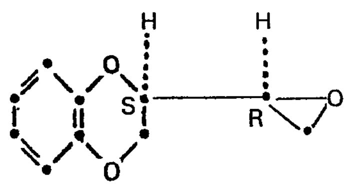

+

(III)

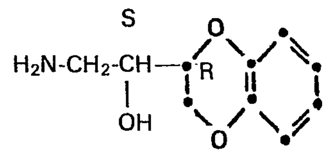

(IV)

ou leurs antipodes ou racémates respectifs et, si nécessaire, on sépare le mélange d'isomères obtenu ou

2) on hydrogénolyse ou on hydrolyse un composé de formule V ou le mélange de diastéréomères érythro qui contient un composé de formule V

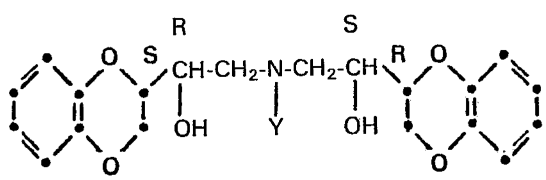

(V)

où Y représente un radical $\alpha$-araliphatique ou un radical acyle; ou on hydrolyse des composés de formule V où Y représente un acyle et l'un des groupes hydroxy est protégé avec un radical acyle, et, si nécessaire, on dédouble le mélange d'isomères obtenu, ou

3) on chauffe un composé érythro de formule III ou un de ses racémates avec de l'ammoniac et, si nécessaire, on dédouble le mélange d'isomères obtenu et, si on le désire, on transforme un composé libre obtenu en un sel d'addition d'acide ou un sel d'addition d'acide obtenu en le composé libre ou en un autre sel d'addition d'acide.

9. Mélange d'isomères de méso- et d,ℓ-3-benzyl-1-(2,3-dihydro-1,4-benzodioxin-2-yl)-5-(2,3-dihydro-1,4-benzodioxin-2-yl)-3-azapentane-1,5-diol de la série érythro, et ses sels d'addition d'acides.

10. Le 3-benzyl-1-(2,3-dihydro-1,4-benzodioxin-2S-yl)-5-(2,3-dihydro-1,4-benzodioxin-2R-yl)-3-azapentane-1R,5S-diol et ses sels d'addition d'acides.

**Revendications pour l'Etat Contractant:** AT

1. Procédé de préparation du nouveau 1-(2,3-dihydro-1,4-benzodioxin-2S-yl)-5-(2,3-dihydro-1,4-benzodioxin-2R-yl)-azapentane-1R,5S-diol de formule

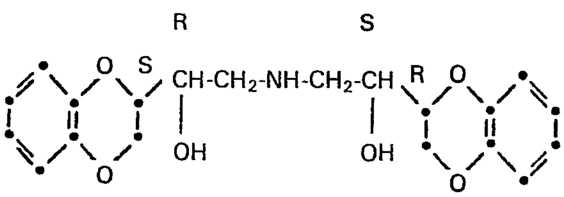

(II),

et de ses sels d'addition d'acides, caractérisé en ce que

1) on fait réagir des composés érythro de formules III et IV

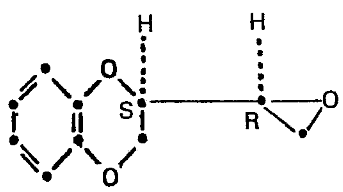

(III) +

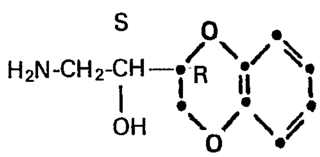

(IV)

ou leurs antipodes ou racémates respectifs, et, si nécessaire, on dédouble le mélange d'isomères obtenu ou

2) on hydrogénolyse ou hydrolyse un composé de formule V ou le mélange de diastéréomères érythro qui contient un composé de formule V

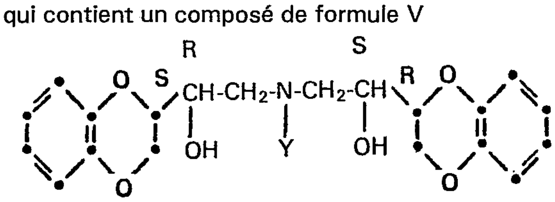

(V)

où Y représente un radical α-araliphatique ou un radical acyle; ou on hydrolyse des composés de formule V où Y représente un acyle et l'un des groupes hydroxy est protégé avec un radical acyle, et, si nécessaire on dédouble le mélange d'isomères, ou

3) on chauffe un composé érythro de formule III ou un de ses racémates avec de l'ammoniac et, si nécessaire, on dédouble le mélange d'isomères obtenu et, si on le désire, on transforme un composé libre obtenu en un sel d'addition d'acide ou un sel d'addition d'acide obtenu en le composé libre ou en un autre sel d'addition d'acide.

2. Composé selon la revendication 1, caractérisé en ce qu'on conduit la réaction 1) à la température de reflux du mélange réactionnel.

3. Procédé selon la revendication 1, caractérisé en ce que dans la réaction 2) on hydrogénolyse un produit de départ de formule V, où Y représente un radical α-araliphatique, par traitement avec de l'hydrogène catalytiquement activé, de l'hydrogène naissant ou de l'hydrogène produit par électrolyse.

4. Procédé selon la revendication 1, caractérisé en ce que dans la réaction 2) d'un produit de départ où Y représente un acyle, on sépare ce radical acyle avec des acides ou des bases aqueux, ou en ce qu'on le séparer avec des hydrures complexes de métaux légers.

5. Procédé selon la revendication 1, caractérisé en ce que dans la réaction 3) on utilise un demi équivalent molaire d'ammoniac.

.6. Procédé selon la revendication 1, caractérisé en ce qu'on dédouble un mélange d'isomères ou de leurs sels obtenu par chromatographie ou cristallisation.

7. Procédé selon l'une des revendications 1 à 6, caractérisé en ce qu'on utilise comme produit de départ un produit intermédiaire que l'on peut obtenir à une étape quelconque du procédé et en ce qu'on réalise les étapes restantes du procédé, ou bien où l'on forme un produit de départ dans les conditions de la réaction ou bien où on l'utilise sous la forme d'un antipode optiquement pur.

8. Procédé selon la revendication 1 de préparation du nouveau 1-(2,3-dihydro-1,4-benzodioxin-2S-yl)-5-(2,3-dihydro-1,4-benzodioxin-2R-yl)-3-azapentane-1R,5S-diol et de ses sels d'addition d'acides, caractérisé en ce que

1) on fait réagir des composés de formules III et IV indiquées dans la revendication 1, ou leurs antipodes, ou

2) on hydrogénolyse ou hydrolyse un composé de formule V présenté dans la revendication 1 et, si on le désire, on transforme un composé libre obtenu en un sel d'addition d'acide ou un sel d'addition d'acide obtenu en le composé libre ou un autre sel d'addition d'acide.

9. Procédé selon la revendication 8, caractérisé en ce qu'on conduit la réaction 1) à la température de reflux du mélange réactionnel.

10. Procédé selon la revendication 8, caractérisé en ce que dans la réaction 2) on hydrogénolyse un produit de départ de formule V, où Y représente un radical α-araliphatique, par traitement avec de l'hydrogène catalytiquement activé, naissant ou produit par électrolyse.

11. Procédé selon la revendication 8, caractérisé en ce que dans la réaction 2) d'un produit de départ, où Y représente un acyle, on sépare ce radical acyle avec des acides ou bases aqueux, ou avec des hydrures de complexes de métaux légers.

12. Procédé selon l'une des revendications 8 à 11, caractérisé en ce qu'on utilise comme produit de départ un produit intermédiaire que l'on peut obtenir à une étape quelconque du procédé et en ce qu'on réalise les étapes restantes du procédé, ou bien où l'on forme un produit de départ dans les conditions de la réaction, ou bien où on l'utilise sous la forme d'un antipode optiquement pur.

13. Procédé de préparation d'une préparation pharmaceutique, caractérisé par le traitement d'une substance active selon l'invention, selon l'une des revendications 1 à 7, avec un support pharmaceutique.

14. Procédé de préparation d'une préparation pharmaceutique, caractérisé par le traitement d'une substance active selon l'invention, selon l'une des revendications 8 à 12, avec un support pharmaceutique.